# EUROPEAN PATENT APPLICATION

(11) **EP 1 568 693 A1**
(43) Date of publication of application: **31.08.2005**
(21) Application number: 03810618.3
(22) Date of filing: 06.11.2003
(51) Int. Cl.: C07D 215/14, C07D 319/06

(54) **PROCESS FOR PRODUCING QUINOLINECARBALDEHYDE**

(30) Priority: 06.11.2002 JP 2002322170; 06.11.2002 JP 2002322172
(71) Applicant: Nissan Chemical Industries, Ltd., Tokyo 101-0054 (JP)
(72) Inventor: NAGASHIMA, Kensuke, c/o KURARAY CO., LTD., Kitakanbara-gun, Niigata 959-2691 (JP); FUKUMOTO, Takashi, c/o KURARAY CO., LTD., Kitakanbara-gun, Niigata 959-2691 (JP); HAYASHIBARA, Tatsuhiko, c/o KURARAY CO., LTD., Kitakanbara-gun, Niigata 959-2691 (JP); TORIHARA, Masahiro, c/o KURARAY CO., LTD., Kitakanbara-gun, Niigata 959-2691 (JP)
(74) Representative: von Kreisler, Alek
(86) International application number: PCT/JP2003/014134
(87) International publication number: WO 2004/041787

(57) **Abstract**

A novel production method of quinolinecarbaldehydes (IV), which includes reacting aminobenzophenones (I) with β-ketoaldehyde derivative (II) in the presence of an acid to give quinolinecarbaldehyde derivative (III), and then hydrolyzing the quinolinecarbaldehyde derivative and a method of producing novel β-ketoaldehyde derivative (II-1), which includes reacting metal alkoxide compound (V) with diol derivative compound (VI) in the presence of an acid.

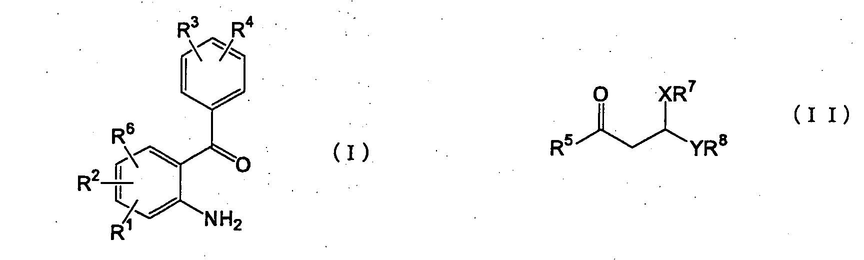

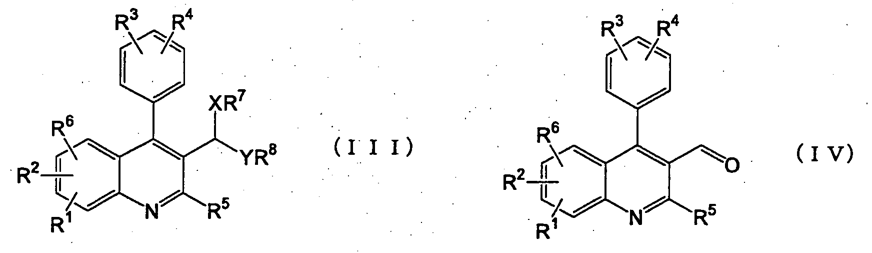

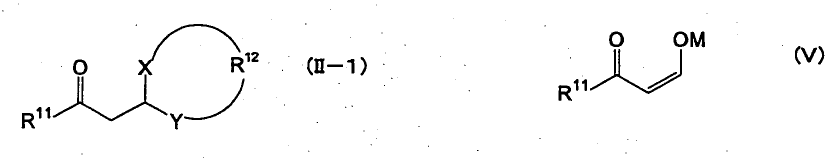

HX-R¹²-YH (VI)

## Description

### Technical Field

The present invention relates to a production method of quinolinecarbaldehydes; a β-ketoaldehyde derivative and a quinolinecarbaldehyde derivative, which are novel starting materials or intermediates for a production method thereof; and a production method of the novel β-ketoaldehyde derivative. The quinolinecarbaldehydes obtained by the present invention are useful as synthetic intermediates for pharmaceutical agents, agricultural chemicals and the like, for example, synthetic intermediates for quinoline type mevalolactone derivatives known as inhibitors of HMG-CoA reductase, which is a rate-limiting enzyme for cholesterol biosynthesis.

### Background Art

Conventionally, as production methods of quinolinecarbaldehydes, such as 2-cyclopropyl-4-(4'-fluorophenyl)quinoline-3-carbaldehyde, there are known (1) a method comprising reducing the corresponding quinolinecarboxylic acid ester with a metal hydride of diisobutyl aluminum hydride and the like to give the corresponding quinolinecarbinol, namely, 4-(4'-fluorophenyl)-2-cyclopropyl-3-hydroxymethylquinoline, and then oxidizing the compound using an oxidant such as pyridinium chlorochromate, oxalylchloride/dimethyl sulfoxide/tertiary amine (Swern oxidation), a sulfur trioxide pyridine complex and the like (e.g., JP-A-01-279866, JP-A-06-329540), and (2) a method comprising oxidizing 4-(4'-fluorophenyl)-2-cyclopropyl-3-hydroxymethylquinoline using a hypohalite salt in a solvent such as dichloromethane and the like in the presence of a nitroxyl radical derivative (e.g., JP-A-08-27114) are known.

However, the above-mentioned method (1) and method (2) both use the corresponding quinolinecarbinol as a starting material for the production of quinolinecarbaldehydes and oxidize the alcohol moiety to aldehyde, wherein the quinolinecarbinol needs to be obtained by reducing the corresponding quinolinecarboxylic acid ester, thus making the steps complicated. In addition, method (1) is associated with a problem of the treatment of effluent containing environmentally harmful chrome ion with regard to the use of pyridinium chlorochromate as an oxidant, and a problem of side production of dimethyl sulfide having excessive odor in the Swern oxidation and oxidation using a sulfur trioxide pyridine complex. In addition, method (2) has problems in that it generally requires environmentally harmful halogenated hydrocarbon such as dichloromethane and the like as a solvent, and the like.

Therefore, these methods are hardly industrially advantageous production methods of quinolinecarbaldehydes, and a method capable of producing efficiently and industrially advantageously in a short step has been desired.

Moreover, as a β-ketoaldehyde derivative, which is one of the starting compounds in the production method of quinolinecarbaldehydes, which is the new invention described in the present specification, for example, 3-cyclopropyl-1,1-diethoxy-3-oxopropane is known, and this compound is produced by reacting sodium formyl cyclopropyl methyl ketone with ethanol in the presence of sulfuric acid (Ukr. Khim. Zh., page 42, No. 4, p. 407 (1976)).

However, this compound is poor in stability and, for example, ethoxy group undergoes β-elimination and enolization under basic conditions, and acetal moiety easily gets hydrolyzed under acidic conditions. Therefore, when this compound is used as a starting material, problems occur in that the compound is decomposed, the yield of the object compound decreases and the like. Thus, a β-ketoaldehyde derivative, which is stable even under acidic conditions, has been desired.

### Disclosure of the Invention

The present inventors have conducted intensive studies and found a method of conveniently and industrially advantageously producing quinolinecarbaldehydes in a short step as compared to conventional methods, and also found a β-ketoaldehyde derivative useful as a starting compound of the production method, which resulted in the completion of the present invention.

### Disclosure of the Invention

Accordingly, the present invention provides the following.
(1) A production method of quinolinecarbaldehydes of the formula (IV) 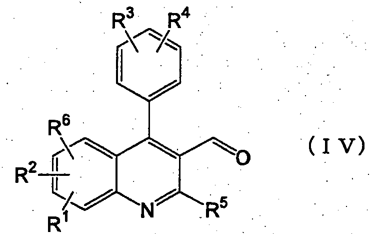 wherein R¹, R², R³, R⁴ and R⁶ are each a hydrogen atom, a halogen atom, an optionally protected hydroxyl group, an optionally substituted alkyl group, an optionally substituted aryl group, an optionally substituted aralkyl group, an optionally substituted alkoxy group, an optionally substituted aryloxy group, or R⁹R¹⁰N- wherein R⁹ and R¹⁰ are each an optionally substituted alkyl group, R¹ and R² are optionally linked to show -CH=CH-CH=CH- and R⁵ is an optionally substituted alkyl group or an optionally substituted aryl group
   [hereinafter to be abbreviated as quinolinecarbaldehydes (IV)], which comprises reacting aminobenzophenones of the formula (I) 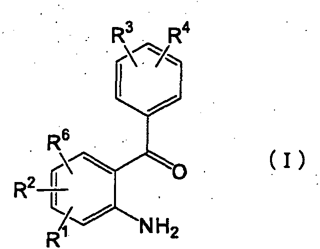 wherein R¹, R², R³, R⁴ and R⁶ are as defined above [hereinafter to be abbreviated as aminobenzophenones (I)] with a β-ketoaldehyde derivative of the formula (II) 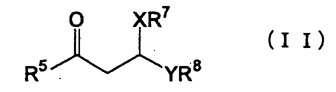 wherein R⁵ is as defined above, R⁷ and R⁸ are each an optionally substituted alkyl group, an optionally substituted acyl group or an optionally substituted aralkyl group, or linked to show an optionally substituted alkylene group, an optionally substituted arylene group or an aralkylene group, and X and Y are the same or different and each is an oxygen atom or a sulfur atom
   [hereinafter to be abbreviated as β-ketoaldehyde derivative (II)] in the presence of an acid to give a quinolinecarbaldehyde derivative of the formula (III) 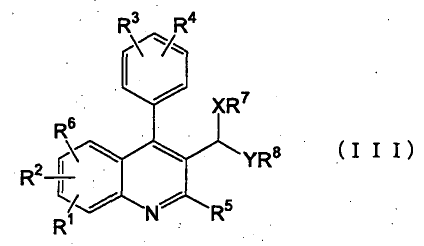 wherein R¹, R², R³, R⁴, R⁵, R⁶, R⁷, R⁸, X and Y are as defined above [hereinafter to be abbreviated as quinolinecarbaldehyde derivative (III)], and then hydrolyzing the quinolinecarbaldehyde derivative (III).
(2) A production method of a quinolinecarbaldehyde derivative (III), which comprises reacting aminobenzophenones (I) with β-ketoaldehyde derivative (II) in the presence of an acid.
(3) A production method of quinolinecarbaldehydes (IV), which comprises hydrolyzing a quinolinecarbaldehyde derivative (III).
(4) The production method of (1), wherein, in each formula, R¹, R², R³ and R⁶ are hydrogen atoms, R⁴ is a halogen atom, R⁵ is an alkyl group having 1 to 6 carbon atoms, R⁷ and R⁸ are linked to show an alkylene group, and X and Y are both oxygen atoms.
(5) The production method of (4), wherein, in each formula, R¹, R², R³ and R⁶ are hydrogen atoms, R⁴ is a fluorine atom, R⁵ is a cyclopropyl group, R⁷ and R⁸ are linked to show an ethylene group, a trimethylene group, a 2-methyltrimethylene group or a 2,2-dimethyltrimethylene group, and X and Y are both oxygen atoms.
(6) A quinolinecarbaldehyde derivative (III), which is an intermediate for the above-mentioned production method.
(7) The quinolinecarbaldehyde derivative (III) of (6), wherein R¹, R², R³ and R⁶ are hydrogen atoms, R⁴ is a halogen atom, R⁵ is an alkyl group having 1 to 6 carbon atoms, R⁷ and R⁸ are linked to show an alkylene group, X and Y are both oxygen atoms.
(8) The quinolinecarbaldehyde derivative (III) of (7), wherein R¹, R², R³ and R⁶ are hydrogen atoms, R⁴ is a fluorine atom, R⁵ is a cyclopropyl group, R⁷ and R⁸ are linked to show an ethylene group, a trimethylene group, a 2-methyltrimethylene group or a 2,2-dimethyltrimethylene group, X and Y are both oxygen atoms.
(9) A β-ketoaldehyde derivative of the formula (II-1) 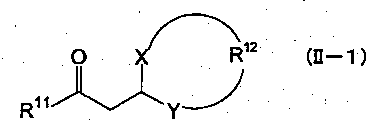 wherein R¹¹ is an optionally substituted alkyl group, R¹² is an optionally substituted alkylene group, an optionally substituted arylene group or an aralkylene group, and X and Y are the same or different and each is an oxygen atom or a sulfur atom [hereinafter to be abbreviated as β-ketoaldehyde derivative (II-1)].
(10) The β-ketoaldehyde derivative (II-1) of (9), wherein R¹² is an optionally substituted alkylene group having 2 to 6 carbon atoms, and X and Y are both oxygen atoms.
(11) The β-ketoaldehyde derivative (II-1) of (10), wherein R¹¹ is an optionally substituted cycloalkyl group, R¹² is an ethylene group, a trimethylene group, a 2-methyltrimethylene group or a 2,2-dimethyltrimethylene group, and X and Y are both oxygen atoms.
(12) The β-ketoaldehyde derivative (II-1) of (9), wherein R¹¹ is an optionally substituted cycloalkyl group, R¹² is an ethylene group, and X and Y are each an oxygen atom or a sulfur atom.
(13) A production method of β-ketoaldehyde derivative (II-1), which comprises reacting a metal alkoxide compound of the formula (V) 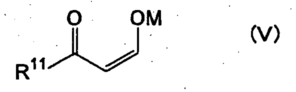 wherein R¹¹ is an optionally substituted alkyl group and M is an alkali metal [hereinafter to be abbreviated as metal alkoxide compound (V)] with a compound of the formula (VI)

   HX-R¹²-YH (VI)

   wherein R¹² is an optionally substituted alkylene group, an optionally substituted arylene group or aralkylene group, and X and Y are the same or different and each is an oxygen atom or a sulfur atom [hereinafter to be abbreviated as compound (VI)] in the presence of an acid.
(14) The production method of (13), wherein R¹¹ is an optionally substituted cycloalkyl group, R¹² is an alkylene group having 2 to 6 carbon atoms, and X and Y are both oxygen atoms.
(15) The production method of (14), wherein R¹¹ is an optionally substituted cycloalkyl group, R¹² is an ethylene group, a trimethylene group, a 2-methyltrimethylene group or a 2,2-dimethyltrimethylene group, and X and Y are both oxygen atoms.
(16) The production method of (13), wherein R¹¹ is an optionally substituted cycloalkyl group, R¹² is an ethylene group, and X and Y are each an oxygen atom or a sulfur atom.

### Best Mode for Carrying Out the Invention

### (Compound)

In each of the above-mentioned formulas, the alkyl group for an of R¹, R², R³, R⁴, R⁵, R⁶, R⁷, R⁸, R⁹, R¹⁰ and R¹¹ may be linear, branched or cyclic. A linear or branched alkyl group preferably having 1 to 6, more preferably 1 to 4, carbon atoms, such as methyl group, ethyl group, n-propyl group, isopropyl group, n-butyl group, isobutyl group, tert-butyl group, pentyl group, isopentyl group, hexyl group and the like, and preferably, a cycloalkyl group having 3 to 6 carbon atoms, such as cyclopropyl group, cyclobutyl group, cyclopentyl group, cyclohexyl group and the like can be mentioned. These alkyl groups may have a substituent, and as such substituent, for example, a hydroxyl group; an alkoxy group preferably having 1 to 4 carbon atoms such as methoxy group, ethoxy group, propoxy group, butoxy group and the like; an optionally substituted aryl group preferably having 6 to 10 carbon atoms such as phenyl group, p-methoxyphenyl group, p-chlorophenyl group and the like; a halogen atom such as fluorine atom, chlorine atom, bromine atom, iodine atom and the like; and the like can be mentioned. In the case of a cycloalkyl group, the cycloalkyl group is optionally further substituted by a linear or branched alkyl group preferably having 1 to 6 carbon atoms such as methyl group, ethyl group, n-propyl group, isopropyl group, n-butyl group, isobutyl group, tert-butyl group, pentyl group, isopentyl group, hexyl group and the like.

As the aryl group for any of R¹, R², R³, R⁴, R⁵ and R⁶, an aryl group preferably having 6 to 10 carbon atoms, such as phenyl group, naphthyl group and the like can be mentioned. As the aralkyl group for any of R¹, R², R³, R⁴, R⁶, R⁷ and R⁸, an aralkyl group having, as the alkyl moiety, an alkyl group preferably having 1 to 6 carbon atoms and as the aryl moiety, an aryl group having 6 to 10 carbon atoms, such as benzyl group, naphthylmethyl group and the like can be mentioned. These aryl groups and aralkyl groups may have a substituent, and as such substituent, for example, a hydroxyl group; an alkyl group preferably having 1 to 6 carbon atoms such as methyl group, ethyl group, n-propyl group, isopropyl group, n-butyl group, isobutyl group, tert-butyl group, n-pentyl group, n-hexyl group and the like; an alkoxy group preferably having 1 to 4 carbon atoms such as methoxy group, ethoxy group, propoxy group, butoxy group and the like; an optionally substituted aryl group preferably having 6 to 10 carbon atoms such as phenyl group, p-methoxyphenyl group, p-chlorophenyl group and the like; a halogen atom such as fluorine atom, chlorine atom, bromine atom, iodine atom and the like; and the like can be mentioned.

As alkoxy group for any of R¹, R², R³, R⁴ and R⁶, a linear or branched alkoxy group preferably having 1 to 4 carbon atoms, such as methoxy group, ethoxy group, propoxy group, butoxy group and the like can be mentioned. These alkoxy groups may have a substituent, and such as substituent, for example, a hydroxyl group; an alkoxy group preferably having 1 to 4 carbon atoms such as methoxy group, ethoxy group, propoxy group, butoxy group and the like; an optionally substituted aryl group preferably having 6 to 10 carbon atoms such as phenyl group, p-methoxyphenyl group, p-chlorophenyl group and the like; a halogen atom such as fluorine atom, chlorine atom, bromine atom, iodine atom and the like; and the like can be mentioned.

As the aryloxy group for any of R¹, R², R³, R⁴ and R⁶, an aryloxy group having, as the aryl moiety, an aryl group preferably having 6 to 10 carbon atoms, such as phenoxy group, naphthyloxy group and the like can be mentioned. These aryloxy groups may have a substituent, and as such substituent, for example, a hydroxyl group; an alkyl group preferably having 1 to 6 carbon atoms such as methyl group, ethyl group, n-propyl group, isopropyl group, n-butyl group, isobutyl group, tert-butyl group, n-pentyl group, n-hexyl group and the like; an alkoxy group preferably having 1 to 4 carbon atoms such as methoxy group, ethoxy group, propoxy group, butoxy group and the like; an aryl group preferably having 6 to 10 carbon atoms, which optionally has a substituent, such as phenyl group, p-methoxyphenyl group, p-chlorophenyl group and the like; a halogen atom such as fluorine atom, chlorine atom, bromine atom, iodine atom and the like; and the like can be mentioned.

As the halogen atom for any of R¹, R², R³, R⁴ and R⁶, for example, fluorine atom, chlorine atom, bromine atom, iodine atom and the like can be mentioned. Of these, fluorine atom is preferable.

The hydroxyl-protecting group of the optionally protected hydroxyl group for any of R¹, R², R³, R⁴ and R⁶ is not particularly limited as long as it is a protecting group generally used for protecting a hydroxyl group and, for example, an aralkyl group such as benzyl group and the like; a tri-substituted silyl group such as trimethylsilyl group, tert-butyldimethylsilyl group, tert-butyldiphenylsilyl group and the like; an ether type protecting group such as methoxymethyl group, 1-ethoxyethyl group, tetrahydrofuranyl group, tetrahydropyranyl group and the like; and the like can be mentioned.

As the acyl group for any of R⁷ and R⁸, for example, an alkylcarbonyl group having, as the alkyl moiety, preferably a linear or branched alkyl group having 1 to 6 carbon atoms, such as acetyl group and the like; an arylcarbonyl group having, as the aryl moiety, preferably an aryl group having 6 to 10 carbon atoms such as benzoyl group and the like; and the like can be mentioned. These acyl groups may have a substituent, and as such substituent, for example, a hydroxyl group; an alkyl group preferably having 1 to 6 carbon atoms such as methyl group, ethyl group, n-propyl group, isopropyl group, n-butyl group, isobutyl group, tert-butyl group, n-pentyl group, n-hexyl group and the like; an alkoxy group preferably having 1 to 4 carbon atoms, such as methoxy group, ethoxy group, propoxy group, butoxy group and the like; an aryl group preferably having 6 to 10 carbon atoms, which optionally has a substituent, such as phenyl group, p-methoxyphenyl group, p-chlorophenyl group and the like; and the like can be mentioned.

As the alkylene group that R⁷ and R⁸ are linked to show, and the alkylene group for R¹², a linear or branched alkylene group preferably having 2 to 6 carbon atoms, such as ethylene group, trimethylene group, tetramethylene group, 2-methyltrimethylene group, pentamethylene group, 2,2-dimethyltrimethylene group and the like can be mentioned. Of these, ethylene group, trimethylene group, 2-methyltrimethylene group and 2,2-dimethyltrimethylene group are particularly preferable. These alkylene groups may have a substituent, and as such substituent, for example, an alkoxy group preferably having 1 to 4 carbon atoms, such as methoxy group, ethoxy group, propoxy group, butoxy group and the like; an aryl group preferably having 6 to 10 carbon atoms, which optionally has a substituent, such as phenyl group, p-methoxyphenyl group, p-chlorophenyl group and the like; a halogen atom such as fluorine atom, chlorine atom, bromine atom, iodine atom and the like; and the like can be mentioned.

As the arylene group that R⁷ and R⁸ are linked to show, and the arylene group for R¹², an arylene group preferably having 6 to 10 carbon atoms, such as o-phenylene group, 2,3-naphthalenediyl group and the like can be mentioned. These arylene groups may have a substituent, and as such substituent, for example, an alkyl group preferably having 1 to 6 carbon atoms such as methyl group, ethyl group, n-propyl group, isopropyl group, n-butyl group, isobutyl group, tert-butyl group, n-pentyl group, n-hexyl group and the like; an alkoxy group preferably having 1 to 4 carbon atoms, such as methoxy group, ethoxy group, propoxy group, butoxy group and the like; an aryl group preferably having 6 to 10 carbon atoms, which optionally has a substituent, such as phenyl group, p-methoxyphenyl group, p-chlorophenyl group and the like; a halogen atom such as fluorine atom, chlorine atom, bromine atom, iodine atom and the like; and the like can be mentioned.

As the alkali metal represented by M, for example, lithium, sodium, potassium and the like can be mentioned.

As the aralkylene group that R⁷ and R⁸ are linked to show, and the aralkylene group for R¹², an aralkylene group preferably having, as the alkylene moiety, an alkylene group having 2 to 6 carbon atoms and preferably having, as the arylene moiety, an arylene group having 6 to 10 carbon atoms, such as 1,2-benzo-2-butene group, 2,3-naphtho-2-butene group and the like can be mentioned.

As the aminobenzophenones (I), preferably a compound wherein R¹, R², R³ and R⁶ are hydrogen atoms and R⁴ is a halogen atom can be mentioned.

As the aminobenzophenones (I), more preferably a compound wherein R¹, R², R³ and R⁶ are hydrogen atoms and R⁴ is a fluorine atom can be mentioned.

As the β-ketoaldehyde derivative (II), preferably a compound wherein R⁵ is an alkyl group having 1 to 6 carbon atoms, X and Y are both oxygen atoms, and R⁷ and R⁸ are linked to show an alkylene group can be mentioned.

Of the β-ketoaldehyde derivatives (II), particularly preferred is a compound wherein R⁵ is an optionally substituted alkyl group and R⁷ and R⁸ are linked to show an optionally substituted alkylene group, an optionally substituted arylene group or an aralkylene group [hereinafter to be abbreviated as β-ketoaldehyde derivative (II-1)].

Moreover, as the β-ketoaldehyde derivative (II-1), particularly preferred is a compound wherein R¹¹ is an optionally substituted cycloalkyl group, R¹² is a group of any of an ethylene group, a trimethylene group, a 2-methyltrimethylene group and a 2,2-dimethyltrimethylene group, and X and Y are both oxygen atoms or each is an oxygen atom or a sulfur atom, such as 2-(2-cyclopropyl-2-oxo-ethyl)-1,3-dioxane, 2-(2-cyclopropyl-2-oxo-ethyl)-5-methyl-1,3-dioxane, 2-(2-cyclopropyl-2-oxo-ethyl)-5,5-dimethyl-1,3-dioxane, 2-(2-cyclopropyl-2-oxo-ethyl)-1,3-dioxolane, 2-(2-cyclopropyl-2-oxo-ethyl)-4-methyl-1,3-dioxolane, 2-(2-cyclopropyl-2-oxoethyl)-1-oxa-3-thiolane, 2-acetonyl-5-methyl-1,3-dioxane, 2-acetonyl-4-methyl-1,3-dioxol,ane, 2-(2-cyclohexyl-2-oxo-ethyl)-5-methyl-1,3-dioxane, 2-(2-cyclohexyl-2-oxo-ethyl)-4-methyl-1,3-dioxolane and the like.

The novel β-ketoaldehyde derivative (II-1) and quinolinecarbaldehyde derivative (III), which is a novel intermediate, of the present invention are not only useful as starting materials or intermediates for the production method of the present invention, which industrially advantageously produces a quinolinecalbaldehyde derivative useful as a synthetic intermediate for a quinoline type mevalolactone derivative known as an inhibitor of HMG-CoA reductase, which is a rate-limiting enzyme for cholesterol biosynthesis, but also can be widely used as synthetic intermediates for various pharmaceutical agents and agricultural chemicals.

### (Production method)

### Step 1: step for obtaining quinolinecarbaldehyde derivative (III) by reacting aminobenzophenones (I) with β-ketoaldehyde derivative (II) in the presence of an acid

Step 1 may be conducted in the presence or absence of a solvent. The usable solvent is not particularly limited as long as the reaction is not adversely affected and, for example, aliphatic hydrocarbons such as hexane, heptane and the like; aromatic hydrocarbons such as toluene, xylene and the like; amides such as dimethylformamide, N-methylpyrrolidone and the like; ethers such as diisopropy ether, tetrahydrofuran and the like; alcohols such as butanol, ethylene glycol, 2-methylpropanediol and the like; and the like can be mentioned. These solvents may be used alone or in a mixture of two or more kinds thereof. While the amount of the solvent to be used is not particularly limited, it is preferably in the range of 2-20 mass times the amount of aminobenzophenones (I) in view of the productivity, economic aspects and the like.

As the acid to be used in Step 1, for example, sulfonic acids such as p-toluenesulfonic acid, methanesulfonic acid and the like; carboxylic acids such as trifluoroacetic acid, chloroacetic acid and the like; mineral acids such as sulfuric acid, hydrochloric acid and the like; Lewis acids such as zinc chloride, titanium chloride and the like; and the like can be mentioned. Of these, sulfonic acids such as p-toluenesulfonic acid, methanesulfonic acid and the like are preferably used. While the amount of the acid to be used is not particularly limited, generally, it is preferably in the range of 0.01-2 mol per 1 mol of aminobenzophenones (I), more preferably in the range of 0.05-1.2 mol per 1 mol of aminobenzophenones (I) in view of the improved economy and selectivity rate. In addition, a salt of these acids with aminobenzophenones (I) can be used as the acid for Step 1.

The amount of the β-ketoaldehyde derivative (II) to be used is generally preferably not less than 0.8 mol per 1 mol of aminobenzophenones (I), and preferably within the range of 0.8-2 mol, particularly preferably within the range of 1-1.5 mol, per 1 mol of aminobenzophenones (I) in view of the economic aspect.

The temperature of Step 1 is preferably within the range of 50-120°C, more preferably within the range of 60-90°C.

In Step 1, water is by-produced along with the reaction. Step 1 may be conducted while removing the water from the reaction system and, for example, a method comprising carrying out the reaction under reduced pressure while evaporating water; a method comprising carrying out the reaction while evaporating water by azeotropic distillation in the coexistence with a solvent that azeotropically reacts with water; a method comprising carrying out the reaction in the presence of molecular sieve, a dehydrating agent such as magnesium sulfate and the like; and the like can be mentioned.

Step 1 can be conducted by, for example, mixing aminobenzophenones (I), β-ketoaldehyde derivative (II), acid and a solvent as necessary and stirring the mixture at a predetermined temperature.

The reaction mixture obtained by Step 1 can be also subjected to the next step mentioned below. Alternatively, water; an aqueous alkali solution such as aqueous sodium hydrogen carbonate solution, aqueous sodium carbonate solution, aqueous sodium hydroxide solution and the like may be added to the reaction mixture to partition the mixture between the organic layer and the aqueous layer, the organic layer is concentrated, the obtained residue is purified as necessary by recrystallization, silica gel column chromatography and the like and used in the Step 2 mentioned below.

β-Ketoaldehyde derivative (II-1), which is a preferable one embodiment of β-ketoaldehyde derivative (II), which is one of the starting materials in Step 1, can be produced by reacting metal alkoxide compound (V) with compound (VI) in the presence of an acid.

As the metal alkoxide compound (V), which is a starting material of this reaction, for example, sodium formyl cyclopropyl methyl ketone and the like can be mentioned, and as compound (VI), which is the other starting material, for example, ethylene glycol, propylene glycol, trimethylene glycol, 2-methyl-1,3-propanediol, 2,2-dimethyl-1,3-propanediol, (1,2-, 1,3- or 2,3-)butanediol, 2-mercaptoethanol and the like can be mentioned.

The reaction is preferably carried out using compound (VI) in excess of, for example, in about 1.5-4 mol times, the amount of metal alkoxide compound (V).

As the acid to be used in this Step, for example, mineral acids such as hydrochloric acid, hydrobromic acid, sulfuric acid, phosphorus acid, perchloric acid and the like; organic acids such as acetic acid, propionic acid, formic acid, p-toluenesulfonic acid, methanesulfonic acid, trifluoroacetic acid, chloroacetic acid, oxalic acid, glycol acid, a hydrate thereof, a salt thereof and the like; a Lewis acid such as boron trifluoride, zinc chloride, titanium chloride and the like; and the like can be mentioned. These acids may be used alone or in a mixture of two or more kinds thereof. The amount of the acid to be used may be any as long as it is not less than an equivalent amount relative to the metal alkoxide compound (V) to be used, and generally, preferably in the range of 1.2-5 equivalents in view of the reaction speed and economic aspect.

The reaction may be carried out in the presence of an organic solvent. While the kind of the organic solvent is not particularly limited as long as it does not affect the reaction, for example, alcohols such as methanol, ethanol, propanol and the like; ethers such as diisopropy ether, dimethoxyethane, tetrahydrofuran and the like; hydrocarbons such as hexane, heptane, octane, toluene, xylene and the like; amides such as dimethylformamide, N-methylpyrrolidone and the like; nitriles such as acetonitrile, benzonitrile and the like; sulfoxides such as dimethyl sulfoxide and the like, or a mixed solvent of these may be used. While the amount of these organic solvents to be used is not particularly limited, it is preferably in the range of not more than 100 mass times the amount of metal alkoxide compound (V) from economical aspect.

While the reaction temperature varies depending on the kind of acid to be used, the kind of solvent and the like, generally, it is preferably in the range of 0-120°C. While the reaction time also varies depending on the reaction temperature, it is generally in the range of 1-10 hr.

As the β-ketoaldehyde derivative (II), for example, 2-(2-cyclopropyl-2-oxo-ethyl)-1,3-dioxane, 2-(2-cyclopropyl-2-oxoethyl)-5-methyl-1,3-dioxane, 2-(2-cyclopropyl-2-oxo-ethyl)-5,5-dimethyl-1,3-dioxane, 2-(2-cyclopropyl-2-oxo-ethyl)-1,3-dioxolane , 2-(2-cyclopropyl-2-oxo-ethyl)-1-oxa-3-thiolane and the like can be mentioned.

### Step 2: step for obtaining quinolinecarbaldehydes (IV) by hydrolyzing quinolinecarbaldehyde derivative (III)

Step 2 is preferably conducted by a method comprising hydrolysis in the coexistence with an acid. As the acid, for example, mineral acids such as hydrochloric acid, sulfuric acid, phosphoric acid, perchloric acid and the like; carboxylic acids such as acetic acid, propionic acid, formic acid, oxalic acid, glycol acid and the like; sulfonic acids such as p-toluenesulfonic acid, methanesulfonic acid and the like, a hydrate thereof, a salt thereof; Lewis acids such as boron trifluoride, zinc chloride and the like; and the like can be mentioned. While the amount of the acid to be used is not particularly limited, it is generally preferably within the range of 0.01-5 mol, more preferably within the range of 1-5 mol, per 1 mol of quinolinecarbaldehyde derivative (III).

While the amount of water to be used in Step 2 is not particularly limited, it is generally preferably not less than 1 mol, more preferably in the range of 1-200 mol, per 1 mol of quinolinecarbaldehyde derivative (III), in view of productivity, economical aspect and the like.

In Step 2, ketone may further coexist in the reaction system to promote the reaction. As the usable ketone, for example, acetone, methyl ethyl ketone, methyl isobutyl ketone and the like can be mentioned. When ketone is made to coexist, its amount of use is not particularly limited, but it is preferably within the range of 0.01-200 mol, more preferably within the range of 1-10 mol, per 1 mol of quinolinecarbaldehyde derivative (III).

Step 2 may be conducted in the presence or absence of a solvent. As the usable solvent, it is not particularly limited as long as the reaction is not adversely affected and, for example, alcohols such as methanol, ethanol, propanol and the like; aliphatic hydrocarbons such as hexane, heptane and the like; aromatic hydrocarbons such as toluene, xylene and the like; amides such as dimethylformamide, N-methylpyrrolidone and the like; ethers such as diisopropy ether, tetrahydrofuran and the like; nitriles such as acetonitrile, benzonitrile and the like; sulfoxides such as dimethyl sulfoxide and the like; and the like can be mentioned. These solvents may be used alone or in a mixture of two or more kinds thereof. While the amount of the solvent to be used is not particularly limited, it is preferably not more than 100 mass times the amount of quinolinecarbaldehyde derivative (III), from the economical aspect. Of these solvents, those permitting layer separation from water may be used for a reaction in a two-layer system.

While the temperature in Step 2 varies depending on the kind of acid to be used, the kind of solvent and the like, generally, it is preferably in the range of 0-120°C. While the reaction time also varies depending on the reaction temperature, it is generally in the range of 1-60 hr.

Step 2 can be conducted by, for example, mixing quinolinecarbaldehyde derivative (III), water, acid and a solvent and ketone as necessary and stirring the mixture at a predetermined temperature.

The thus-obtained quinolinecarbaldehydes (IV) can be isolated and purified from the reaction mixture by a method generally used for the isolation and purification of organic compounds. For example, water; an alkali aqueous solution such as aqueous sodium hydrogen carbonate solution, aqueous sodium carbonate solution, aqueous sodium hydroxide solution and the like are added to the reaction mixture to partition the mixture between the organic layer and the aqueous layer, the organic layer is concentrated and the obtained residue is purified as necessary by recrystallization, silica gel column chromatography and the like.

The metal alkoxide compound (V) such as sodium formyl cyclopropyl methyl ketone used as a starting material in the present invention can be synthesize by a method described in, for example, JP-A-49-124073.

### (Examples)

The present invention is explained in detail in the following by referring to Examples, which are not to be construed as limitative.

### Example 1

Under a nitrogen atmosphere, 1,3-propanediol (31.8 g, 417 mmol) and sulfuric acid (31.28 g, 312 mmol) were added to a four-neck flask (interior content 200 ml) equipped with a stirrer, a thermometer and a funnel for dropwise addition, then a solution of sodium formyl cyclopropyl methyl ketone (27.96 g, 208.5 mmol) in methanol (20 g) was added dropwise with stirring at an internal temperature of 25°C over 1 hr. After the completion of the dropwise addition, the internal temperature was raised to 60°C and the mixture was further stirred for 1 hr. The obtained reaction mixture was added to a saturated aqueous sodium hydrogen carbonate solution (40 g) and the mixture was partitioned between the organic layer and the aqueous layer. The obtained organic layer was analyzed by gas chromatography. As a result, 2-(2-cyclopropyl-2-oxoethyl)-1,3-dioxane (27.0 g) was contained (yield 76%). This organic layer was concentrated and the residue was distilled at 50 Pa to give 2-(2-cyclopropyl-2-oxo-ethyl)-1,3-dioxane (26.22 g) from a fraction at 82-83°C.
2-(2-cyclopropyl-2-oxo-ethyl)-1,3-dioxane
¹H-NMR(300MHz, CDCl₃, TMS, ppm) δ:0.82-0.89(m,2H), 0.99-1.04(m,2H), 1.88-1.95(m,1H), 1.96-2.12(m,1H),
2.80(dd,J=1.4Hz,5.0Hz,2H), 3.78(t,J=12.0Hz,2H),
4.06(dd,J=5.0Hz,J=12.0Hz,2H), 4.97(t,J=5.0Hz,1H)

### Example 2

Under a nitrogen atmosphere, 2-methyl-1,3-propanediol (26.86 g, 298 mmol) and sulfuric acid (8.2 g, 82 mmol) were added to a four-neck flask (interior content 100 ml) equipped with a stirrer, a thermometer and a funnel for dropwise addition, then a solution of sodium formyl cyclopropyl methyl ketone (10 g, 74.6 mmol) in methanol (20 g) was added dropwise with stirring at an internal temperature of 25°C over 1 hr. After the completion of the dropwise addition, the internal temperature was raised to 60°C and the mixture was further stirred for 1 hr. The obtained reaction mixture was added to a saturated aqueous sodium hydrogen carbonate solution (40 g) and the mixture was partitioned between the organic layer and the aqueous layer. The obtained organic layer was analyzed by gas chromatography. As a result, 2-(2-cyclopropyl-2-oxoethyl)-5-methyl-1,3-dioxane (9.92 g) was contained (yield 72%). This organic layer was concentrated and the residue was distilled at 50 Pa to give 2-(2-cyclopropyl-2-oxo-ethyl)-5-methyl-1,3-dioxane (9.11 g) from a fraction at 91-92°C. trans-2-(2-cyclopropyl-2-oxo-ethyl)-5-methyl-1,3-dioxane
¹H-NMR(300MHz, CDCl₃, TMS, ppm) δ:0.70(d,J=6.6Hz,3H), 0.86-0.92(m,2H), 1.04-1.09(m,2H), 1.95-2.00(m,1H),
2.86(d,J=4.4Hz,2H), 3.26(t,J=11.6Hz,2H),
4.03(dd,J=11.8Hz,4.7Hz,2H), 4.92(t,J=5.2Hz,1H)
cis-2-(2-cyclopropyl-2-oxo-ethyl)-5-methyl-1,3-dioxane
¹H-NMR(300MHz, CDCl₃, TMS, ppm) δ:0.86-0.92(m,2H), 1.04-1.09(m,2H), 1.28,(d,J=6.6Hz,3H), 2.00-2.15(m,1H),
2.86(d,J=4.4Hz,2H), 3.79(d,J=11.4Hz,1H), 3.96(d,J=11.4Hz,2H), 4.99(t,J=5.2Hz,1H)

### Example 3

Under a nitrogen atmosphere, ethylene glycol (17.88 g, 298 mmol) and sulfuric acid (8.2 g, 82 mmol, 1.1 mol-fold) were added to a four-neck flask (interior content 100 ml) equipped with a stirrer, a thermometer and a funnel for dropwise addition, then a solution of sodium formyl cyclopropyl methyl ketone (10 g, 74.6 mmol) in methanol (20 g) was added dropwise with stirring at an internal temperature of 25°C over 1 hr. After the completion of the dropwise addition, the internal temperature was raised to 60°C and the mixture was further stirred for 1 hr. The obtained reaction mixture was added to a saturated aqueous sodium hydrogen carbonate solution (40 g) and the mixture was partitioned between the organic layer and the aqueous layer. The obtained organic layer was analyzed by gas chromatography. As a result, 2-(2-cyclopropyl-2-oxo-ethyl)-1,3-dioxolane (8.77 g) was contained (yield 75%). This organic layer was concentrated and the residue was distilled at 50 Pa to give 2-(2-cyclopropyl-2-oxoethyl)-1,3-dioxolane (8.24 g) from a fraction at 93-96°C. 2-(2-cyclopropyl-2-oxo-ethyl)-1,3-dioxolane
¹H-NMR(300MHz, CDCl₃, TMS, ppm) δ:0.88-0.94(m,2H), 1.03-1.13(m,2H), 1.96-2.04(m,1H), 2.92(d,J=5.0Hz,2H), 3.81-4.00(m,4H), 5.28(t,J=5.0Hz,1H)

### Example 4

Under a nitrogen atmosphere, propylene glycol (31.8 g, 417 mmol, 2 mol-fold) and sulfuric acid (31.28 g, 312 mmol, 1.5 mol-fold) were added to a four-neck flask (interior content 200 ml) equipped with a stirrer, a thermometer and a funnel for dropwise addition, then a solution of sodium formyl cyclopropyl methyl ketone (27.96 g, 208.5 mmol) in methanol (20 g) was added dropwise with stirring at an internal temperature of 25°C over 1 hr. After the completion of the dropwise addition, the internal temperature was raised to 60°C and the mixture was further stirred for 1 hr. The obtained reaction mixture was added to a saturated aqueous sodium hydrogen carbonate solution (40 g) and the mixture was partitioned between the organic layer and the aqueous layer. The obtained organic layer was analyzed by gas chromatography. As a result, 2-(2-cyclopropyl-2-oxo-ethyl)-1,3-dioxane (27.0 g) was contained (yield 76%). This organic layer was concentrated and the residue was distilled at 50 Pa to give 2-(2-cyclopropyl-2-oxo-ethyl)-1,3-dioxane (26.22 g) from a fraction at 82-83°C.
2-(2-cyclopropyl-2-oxo-ethyl)-1,3-dioxane
¹H-NMR(300MHz, CDCl₃, TMS, ppm) δ:0.82-0.89(m,2H), 0.99-1.04(m,2H), 1.88-1.95 (m,1H), 1.96-2.12(m,1H),
2.80(dd,J=1.4Hz,J=5Hz,2H), 3.78(t,J=12Hz,2H), 4.06(dd,J=5Hz,J=12Hz,2H), 4.97(t,J=5.0Hz,1H)

### Example 5

Under a nitrogen atmosphere, mercapto ethanol (46.8 g, 400 mmol, 2 mol-fold) and sulfuric acid (30.0 g, 300 mmol, 1.5 mol-fold) were added to a four-neck flask (interior content 300 ml) equipped with a stirrer, a thermometer and a funnel for dropwise addition, then a solution of sodium formyl cyclopropyl methyl ketone (26.8 g, 200 mmol) in methanol (60 g) was added dropwise with stirring at an internal temperature of 25°C over 1 hr. After the completion of the dropwise addition, the internal temperature was raised to 60°C and the mixture was further stirred for 1 hr. The obtained reaction mixture was added to a saturated aqueous sodium hydrogen carbonate solution (40 g) and methyl chloride was added to partition the obtained mixture between the organic layer and the aqueous layer. The obtained organic layer was further extracted 3 times with methylene chloride, combined with the organic layer obtained earlier. The mixture was concentrated and the residue was distilled at 50 Pa to give 2-(2-cyclopropyl-2-oxo-ethyl)-1-oxa-3-thiolane from a fraction at 99°C (8.96 g, yield 28%).
2-(2-cyclopropyl-2-oxo-ethyl)-1-oxa-3-thiolane ¹H-NMR(300MHz, CDCl₃, TMS, ppm) δ:0.88-0.96(m,2H), 1.06-1.10(m,2H), 1.93-2.00(m,1H), 2.96-3.06(m,4H),
2.26(dd,J=6.0Hz,J=17Hz,1H), 3.72-3.89(m,2H), 4.30-4.37(m,1H),5.43(t,J=9.0Hz,1H)

### Example 6

(a) 2-Amino-4'-fluorobenzophenone (2.15 g, 10 mmol), 2-(2-cyclopropyl-2-oxo-ethyl)-1,3-dioxane (1.56 g, 12 mmol) obtained in Example 1, methanesulfonic acid (290 mg, 3 mmol) and toluene (9.69 g) were placed in a flask (interior content 200 ml) equipped with a Dean-Stark trap and the mixture was stirred under the conditions of 0.25 MPa, 70-75°C for 10 hr. During this period, water by-produced with the progress of the reaction was removed from the reaction system by evaporation by azeotropic distillation with toluene. The obtained reaction mixture was allowed to return to room temperature, normal pressures, 5 mass % aqueous sodium carbonate solution (41.2 g, 51.5 mmol) was added dropwise, and toluene (40.5 g) was added to partition the mixture between the organic layer and the aqueous layer. The obtained organic layer was analyzed by gas chromatography to give 2-[2'-cyclopropyl-4'-(4"-fluorophenyl)quinolin-3'-yl]-1,3-dioxane (2.16 g, yield 68.5%). 2-[2'-cyclopropyl-4'-(4"-fluorophenyl)quinolin-3'-yl]-1,3-dioxane
   ¹H-NMR(300MHz, CDCl₃, TMS, ppm) δ:0.9-1.05(m,2H), 1.36-1.41(m,2H), 2.55-2.68(m,1H), 3.80-3.94(m,1H), 4.00-4.15(m,2H), 5.77(d,J=1.3Hz,1H), 7.10-7.42(m,8H), 7.63(dd,J=6.6Hz,8.3Hz,1H), 7.95(d,J=8.3Hz,1H)
(b) To the organic layer obtained in the above-mentioned (a) was added 2 mass % of hydrochloric acid (20 g), and the mixture was stirred at 40°C for 10 hr. To the obtained reaction mixture was added 5 mass % of aqueous sodium carbonate solution (41.2 g, 51.5 mmol) to partition the mixture between the organic layer and the aqueous layer. The obtained organic layer was analyzed by gas chromatography. As a result, 2-cyclopropyl-4-(4'-fluorophenyl)quinoline-3-carbaldehyde was contained (1.96 g, yield 93.%).

### Example 7

2-Amino-4'-fluorobenzophenone (2.15 g, 10 mmol), 2-(2-cyclopropyl-2-oxo-ethyl)-5-methyl-1,3-dioxane (2.29 g, 12 mmol) obtained in Example 2, methanesulfonic acid (290 mg, 3 mmol) and toluene (9.69 g) were placed in a flask (interior content 200 ml) equipped with a Dean-Stark trap and the mixture was stirred under the conditions of 0.35 MPa, 80-85°C for 10 hr. During this period, water by-produced with the progress of the reaction was removed from the reaction system by evaporation by azeotropic distillation with toluene. The obtained reaction mixture was allowed to return to room temperature, normal pressures, 5 mass % aqueous sodium carbonate solution (41.2 g, 51.5 mmol) was added dropwise, and toluene (40.5 g) was added to partition the mixture between the organic layer and the aqueous layer. The obtained organic layer was analyzed by gas chromatography to give 2-[2'-cyclopropyl-4'-(4"-fluorophenyl)quinolin-3'-yl]-5-methyl-1,3-dioxane (2.73 g, yield 75.4%).
trans-2-[2'-cyclopropyl-4'-(4"-fluorophenyl)quinolin-3'-yl]-5-methyl-1,3-dioxane
¹H-NMR(300MHz, CDCl₃, TMS, ppm) δ:0.70(d,J=6.6Hz,3H), 1.01-1.09(m,2H), 1.36-1.41 (m,2H), 2.15-2.30(m,1H),
3.20(t,J=11.6Hz,1H), 3.24-3.30(m,1H), 4.00-4.15(m,2H),
5.37(s,1H), 7.18-7.42(m,8H), 7.55-7.62(m,1H), 7.92(d,J=8.3Hz,1H)
cis-2-[2'-cyclopropyl-4'-(4"-fluorophenyl)quinolin-3'-yl]-5-methyl-1,3-dioxane
¹H-NMR(300MHz, CDCl₃, TMS, ppm) δ:1.02-1.09(m,2H), 1.36-1.41(m,2H), 1.56 (d,J=6.6Hz,3H), 3.34-3.38(m,1H),
3.80(d,J=9.7Hz,1H), 3.90(d,J=9.7Hz,1H), 4.05-4.15(m,2H),
5.43(s,1H), 7.18-7.42(m,8H), 7.55-7.62(m,1H),
7.93(d,J=8.3Hz,1H)

### Industrial Applicability

According to the production method of the present invention, quinolinecarbaldehydes useful as synthetic intermediates for pharmaceutical agents, agricultural chemicals and the like, for example, quinolinecalbaldehydes such as 2-cyclopropyl-4-(4'-fluorophenyl)quinoline-3-carbaldehyde and the like useful as synthetic intermediates for quinoline type mevalolactone derivatives known as inhibitors of HMG-CoA reductase, which is a rate-limiting enzyme for cholesterol biosynthesis, can be produced efficiently and industrially advantageously by short and simple steps as compared to conventional methods.

In addition, since the novel β-ketoaldehyde derivative (II-1) in the present invention is stable even under acidic conditions, it is not only particularly advantageous as a starting material for the above-mentioned production method performed under acidic conditions but also can be widely used as synthetic intermediates for various pharmaceutical agents and agricultural chemicals, along with the quinolinecarbaldehyde derivative (III), which is a novel intermediate in the present invention.

This application is based on patent application Nos. 2002-322170 and 2002-322172 filed in Japan, the contents of which are hereby incorporated by reference.

## Claims

1. A production method of quinolinecarbaldehyde of the formula (IV) 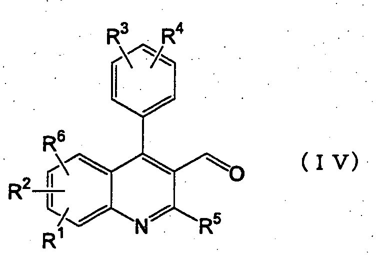 wherein R¹, R², R³, R⁴ and R⁶ are each a hydrogen atom, a halogen atom, an optionally protected hydroxyl group, an optionally substituted alkyl group, an optionally substituted aryl group, an optionally substituted aralkyl group, an optionally substituted alkoxy group, an optionally substituted aryloxy group, or R⁹R¹⁰N- wherein R⁹ and R¹⁰ are each an optionally substituted alkyl group, R¹ and R² are optionally linked to show -CH=CH-CH=CH- and R⁵ is an optionally substituted alkyl group or an optionally substituted aryl group, which comprises reacting aminobenzophenone of the formula (I) 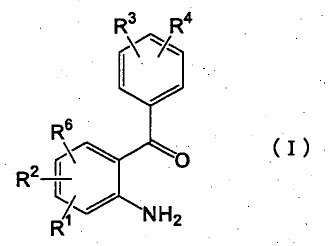 wherein R¹, R², R³, R⁴ and R⁶ are as defined above, with a β-ketoaldehyde derivative of the formula (II) 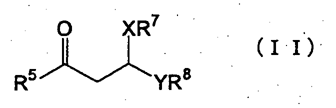 wherein R⁵ is as defined above, R⁷ and R⁸ are each an optionally substituted alkyl group, an optionally substituted acyl group or an optionally substituted aralkyl group, or linked to show an optionally substituted alkylene group, an optionally substituted arylene group or an aralkylene group, and X and Y are the same or different and each is an oxygen atom or a sulfur atom, in the presence of an acid to give a quinolinecarbaldehyde derivative of the formula (III) 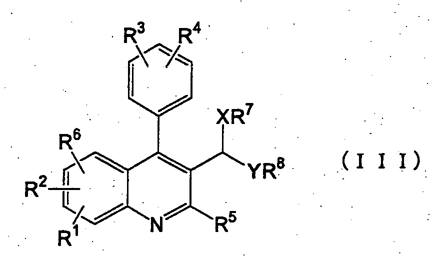 wherein R¹, R², R³, R⁴, R⁵, R⁶, R⁷, R⁸, X and Y are as defined above, and then hydrolyzing said quinolinecarbaldehyde derivative.

2. A production method of a quinolinecarbaldehyde derivative of the formula (III) 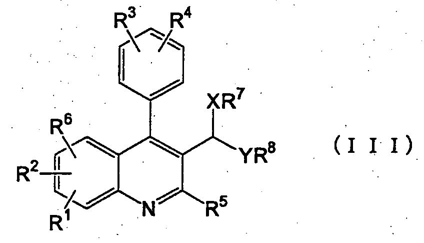 wherein R¹, R², R³, R⁴ and R⁶ are each a hydrogen atom, a halogen atom, an optionally protected hydroxyl group, an optionally substituted alkyl group, an optionally substituted aryl group, an optionally substituted aralkyl group, an optionally substituted alkoxy group, an optionally substituted aryloxy group, or R⁹R¹⁰N- wherein R⁹ and R¹⁰ are each an optionally substituted alkyl group, R¹ and R² are optionally linked to show -CH=CH-CH=CH-, R⁵ is an optionally substituted alkyl group or an optionally substituted aryl group, R⁷ and R⁸ are each an optionally substituted alkyl group, an optionally substituted acyl group or an optionally substituted aralkyl group, or linked to show an optionally substituted alkylene group, an optionally substituted arylene group or an aralkylene group, and X and Y are the same or different and each is an oxygen atom or a sulfur atom, which comprises reacting aminobenzophenone of the formula (I) 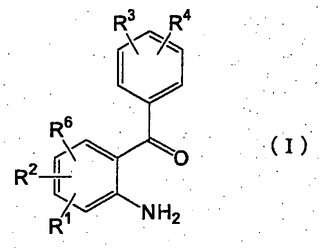 wherein R¹, R², R³, R⁴ and R⁶ are as defined above, with a β-ketoaldehyde derivative of the formula (II) 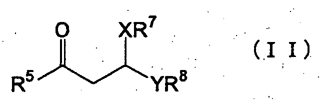 wherein R⁵, R⁷ and R⁸ are as defined above, in the presence of an acid.

3. A production method of quinolinecarbaldehyde of the formula (IV) 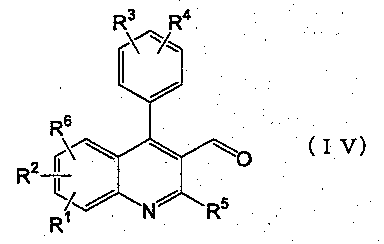 wherein R¹, R², R³, R⁴ and R⁶ are each a hydrogen atom, a halogen atom, an optionally protected hydroxyl group, an optionally substituted alkyl group, an optionally substituted aryl group, an optionally substituted aralkyl group, an optionally substituted alkoxy group, an optionally substituted aryloxy group, or R⁹R¹⁰N- wherein R⁹ and R¹⁰ are each an optionally substituted alkyl group, R¹ and R² is optionally linked to show -CH=CH-CH=CH- and R⁵ is an optionally substituted alkyl group or an optionally substituted aryl group, which comprises hydrolyzing a quinolinecarbaldehyde derivative of the formula (III) 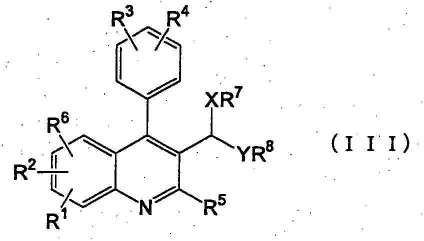 wherein R¹, R², R³, R⁴, R⁵ and R⁶ are as defined above, R⁷ and R⁸ are each an optionally substituted alkyl group, an optionally substituted acyl group or an optionally substituted aralkyl group; or linked to show an optionally substituted alkylene group, an optionally substituted arylene group or an aralkylene group, and X and Y are the same or different and each is an oxygen atom or a sulfur atom.

4. The production method of claim 1, wherein, in each formula, R¹, R², R³ and R⁶ are hydrogen atoms, R⁴ is a halogen atom, R⁵ is an alkyl group having 1 to 6 carbon atoms, R⁷ and R⁸ are linked to show an alkylene group, and X and Y are both oxygen atoms.

5. The production method of claim 4, wherein, in each formula, R¹, R², R³ and R⁶ are hydrogen atoms, R⁴ is a fluorine atom, R⁵ is a cyclopropyl group, R⁷ and R⁸ are linked to show an ethylene group, a trimethylene group, a 2-methyltrimethylene group or a 2,2-dimethyltrimethylene group, and X and Y are both oxygen atoms.

6. A quinolinecarbaldehyde derivative of the formula (III) 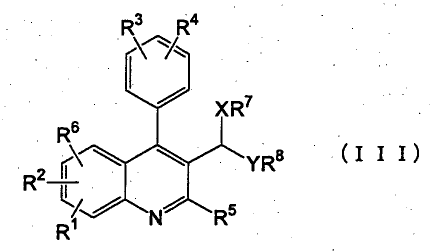 wherein R¹, R², R³, R⁴ and R⁶ are each a hydrogen atom, a halogen atom, an optionally protected hydroxyl group, an optionally substituted alkyl group, an optionally substituted aryl group, an optionally substituted aralkyl group, an optionally substituted alkoxy group, an optionally substituted aryloxy group, or R⁹R¹⁰N- wherein R⁹ and R¹⁰ are each an optionally substituted alkyl group, R¹ and R² are optionally linked to show -CH=CH-CH=CH-, R⁵ is an optionally substituted alkyl group or an optionally substituted aryl group, R⁷ and R⁸ are each an optionally substituted alkyl group, an optionally substituted acyl group or an optionally substituted aralkyl group, or linked to show an optionally substituted alkylene group, an optionally substituted arylene group or an aralkylene group, and X and Y are the same or different and each is an oxygen atom or a sulfur atom.

7. The quinolinecarbaldehyde derivative of claim 6, wherein R¹, R², R³ and R⁶ are hydrogen atoms, R⁴ is a halogen atom, R⁵ is an alkyl group having 1 to 6 carbon atoms, R⁷ and R⁸ are linked to show an alkylene group, and X and Y are both oxygen atoms.

8. The quinolinecarbaldehyde derivative of claim 7, wherein R¹, R², R³ and R⁶ are hydrogen atoms, R⁴ is a fluorine atom, R⁵ is a cyclopropyl group, R⁷ and R⁸ are linked to show an ethylene group, a trimethylene group, a 2-methyltrimethylene group or a 2,2-dimethyltrimethylene group, and X and Y are both oxygen atoms.

9. A β-ketoaldehyde derivative of the formula (II-1) 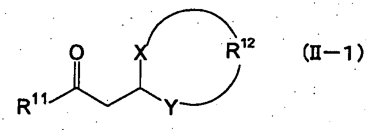 wherein R¹¹ is an optionally substituted alkyl group, R¹² is an optionally substituted alkylene group, an optionally substituted arylene group or an aralkylene group, and X and Y are the same or different and each is an oxygen atom or a sulfur atom.

10. The β-ketoaldehyde derivative of claim 9, wherein R¹² is an optionally substituted alkylene group having 2 to 6 carbon atoms, and X and Y are both oxygen atoms.

11. The β-ketoaldehyde derivative of claim 10, wherein R¹¹ is an optionally substituted cycloalkyl group, R¹² is an ethylene group, a trimethylene group, a 2-methyltrimethylene group or a 2,2-dimethyltrimethylene group, and X and Y are both oxygen atoms.

12. The (-ketoaldehyde derivative of claim 9, wherein R11 is an optionally substituted cycloalkyl group, R12 is an ethylene group, and X and Y are each an oxygen atom or a sulfur atom.

13. A production method of a (-ketoaldehyde derivative of the formula (II-1), which comprises reacting a metal alkoxide compound of the formula (V) 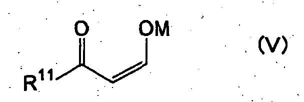 wherein R11 is an optionally substituted alkyl group and M is an alkali metal, with a compound of the formula (VI)
HX-R¹²-YH (VI)
wherein R12 is an optionally substituted alkylene group, an optionally substituted arylene group or aralkylene group, and X and Y are the same or different and each is an oxygen atom or a sulfur atom, in the presence of an acid.

14. The production method of claim 13, wherein R11 is an optionally substituted cycloalkyl group, R12 is an alkylene group having 2 to 6 carbon atoms, and X and Y are both oxygen atoms.

15. The production method of claim 14, wherein R11 is an optionally substituted cycloalkyl group, R12 is an ethylene group, a trimethylene group, a 2-methyltrimethylene group or a 2,2-dimethyltrimethylene group, and X and Y are both oxygen atoms.

16. The production method of claim 13, wherein R11 is an optionally substituted cycloalkyl group, R12 is an ethylene group, and X and Y are each an oxygen atom or a sulfur atom.
